(19) Europäisches Patentamt
European Patent Office
Office européen des brevets

(11) **EP 4 082 430 A1**

(12) **EUROPEAN PATENT APPLICATION**

(43) Date of publication:
**02.11.2022 Bulletin 2022/44**

(21) Application number: **21171027.2**

(22) Date of filing: **28.04.2021**

(51) International Patent Classification (IPC):
**A61B 5/022** $^{(2006.01)}$

(52) Cooperative Patent Classification (CPC):
**A61B 5/02225; A61B 5/7264**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **FRANCK, Christoph Florian**
**5656 AE Eindhoven (NL)**

(74) Representative: **Philips Intellectual Property & Standards**
**High Tech Campus 52**
**5656 AG Eindhoven (NL)**

(54) **DETERMINING BLOOD PRESSURE**

(57) According to an aspect, there is provided a computer-implemented method of determining blood pressure of a subject. The method comprises receiving (101) an envelope data set for the subject. The envelope data set comprises measurements of blood flow in a body part of the subject for respective different degrees of restriction applied to the body part. Next the method comprises using (103) a set of probability density functions, PDFs, to determine respective likelihoods for obtaining the measurements in the envelope data set for different pairs of values of the systolic blood pressure and diastolic blood pressure. Finally, the method comprises determining (105) the systolic blood pressure and the diastolic blood pressure for the subject as the pair of values of the systolic blood pressure and diastolic blood pressure that provides the highest likelihood for the envelope data set.

Fig. 3

EP 4 082 430 A1

**Description**

FIELD OF THE INVENTION

This disclosure relates to determining blood pressure of a subject, and in particular to a computer-implemented method, a computer program product and an apparatus for determining blood pressure from blood flow measurements.

BACKGROUND OF THE INVENTION

A common method for non-invasively measuring blood pressure involves temporarily restricting the arterial blood flow in an extremity (e.g. an arm or leg) by applying external pressure, and then determining a measure of the blood flow when the amount of pressure exerted on the extremity changes. If the external pressure is high enough to completely occlude the arteries even at the peak of the systolic blood pressure, no blood flow will be present in the part of the extremity distal to the site of the restriction. If the external pressure is high enough to occlude the arteries during part of the cardiac cycle, the blood flow through the extremity will be highly pulsatile and also turbulent instead of laminar. If the external pressure is too low to occlude the arteries at any point, the blood flow will be laminar and moderately pulsatile. The restriction of the blood flow is commonly achieved by applying an inflatable cuff around one of the patient's limbs and then inflating and deflating it to achieve the desired degrees of blood flow restriction.

There are at least three methods for determining the blood flow as the degree of restriction changes. The most common method is oscillometry, which works by detecting and measuring the small variations in pressure in the cuff caused by changes in artery volume due to the blood flow. This method is frequently used as it is inexpensive and does not require any sensors or electrical connections in the cuff-the pressure sensor can be placed in the blood pressure monitor along with the pump used to inflate the cuff. A drawback is that this method is sensitive to artefacts caused by motion of the patient, as even small external disturbances of the cuff will cause pressure changes inside that are in the same order of magnitude as the oscillations caused by blood flow.

Another commonly-used method is photoplethysmography (PPG), which measures minute changes in light absorption caused by blood flow in the distal part of the limb using a light source and a photoreceptor. This method is more robust in the presence of motion artefacts, but requires additional connections for the light source and the light sensor.

A third commonly-used method uses a microphone to detect the sounds caused by turbulent flow in the blood vessels, the so-called Korotkoff sounds. These sounds give a very good indication to when the degree of restriction is just large enough to occlude the artery part of the time, but are also susceptible to ambient noise. This method is prevalent when performing manual non-invasive blood pressure measurements using a cuff, a pressure sensor/gauge, and a stethoscope for picking up the Korotkoff sounds.

Other methods for determining blood flow include using Doppler ultrasound to measure the flow velocity in an artery.

In order to determine a patient's blood pressure, the degree of pulsatility of the blood flow needs to be measured at different degrees of restriction (i.e. at different values of the cuff pressure when an inflatable cuff is used). This provides a measurement set containing pairs of values for the degree of restriction and the corresponding measure of blood flow. In the case of an oscillometric measurement, this would be pairs of static cuff pressure and cuff pressure oscillation amplitude (static cuff pressure, cuff pressure oscillation amplitude). The measurement set of value pairs is referred to herein as an "envelope data set" or a "measured envelope". In the case of an envelope data set that includes oscillometric measurements, the envelope data set can be referred to as an oscillation table.

In conventional techniques, once the extent of the envelope is sufficient (i.e. blood flow measurements have been obtained for a range of degrees of restriction), values of systolic and diastolic blood pressure can be estimated from the envelope data set.

A currently-used method for calculating blood pressure values from measured envelope data works by finding reference points in the envelope using interpolation, and deriving the blood pressure from the location of the reference point, for example as described in US 4,984,577 ("Oscillometric non-invasive method for measuring blood pressure and apparatus for automated oscillometric blood pressure measuring"). Another method fits a model function to the set of envelope points and derives the blood pressure values from the parameters obtained by the curve fitting operation, for example as described in US 5,704,362 ("Method for oscillometric blood pressure determination employing curve fitting").

SUMMARY OF THE INVENTION

One drawback of the conventional methods is that they tend to place a large reliance on a few points of the measured envelope, particularly the maximum value of the blood flow, and discard the information contained other points. In particular, the blood pressure calculation is commonly highly dependent on the amplitude and position of the maximum of the measured envelope. As a result, a single erroneous measurement (particularly an erroneous maximum amplitude) can potentially effect a large change in the calculated blood pressure values. This means that the methods tend not to be robust when encountering erroneous envelope points caused by motion artefacts or similar factors.

Another drawback is that some current methods do not have a way to incorporate prior information into the blood pressure calculation. Prior information can include knowledge that certain blood pressure ranges are more likely than others (e.g. the diastolic blood pressure is more likely to be in the 70... 90 mmHg range than outside this range), that certain pulse pressure values are more likely than others, or that the patient's blood pressure is more likely to be close to previous measurements rather than significantly different.

Therefore, there is a need for an improved technique for determining systolic blood pressure and diastolic blood pressure from an envelope data set that is less reliant on particular measurement points or a single measurement point in the envelope data set.

Aspects and embodiments of the disclosed techniques are set out below.

It will be noted that some embodiments also provide a way to include one or more types of prior information in the calculation of the blood pressure. In these embodiments, the prior information can be taken into account by using a maximum a posteriori estimation approach from Bayesian statistics.

According to a first specific aspect, there is provided a computer-implemented method of determining blood pressure of a subject. The method comprises receiving an envelope data set for the subject, the envelope data set comprising measurements of blood flow in a body part of the subject for respective different degrees of restriction applied to the body part; using a set of probability density functions, PDFs, to determine respective likelihoods for obtaining the measurements in the envelope data set for different pairs of values of the systolic blood pressure and diastolic blood pressure; and determining the systolic blood pressure and the diastolic blood pressure for the subject as the pair of values of the systolic blood pressure and diastolic blood pressure that provides the highest likelihood for the envelope data set. Thus, the first aspect provides a technique for determining systolic blood pressure and diastolic blood pressure measurement that is less reliant on particular measurement points or a single measurement point in the envelope data set. As a result, the technique is less susceptible to errors arising from outlier measurements and other errors in the envelope data set.

In some embodiments, the step of using the set of PDFs comprises, for each measurement in the envelope data set, determining respective probabilities for obtaining said measurement in the envelope data set for different pairs of values of the systolic blood pressure and diastolic blood pressure; and, for each pair of values of the systolic blood pressure and diastolic blood pressure, determining the likelihood for obtaining the measurements in the envelope data set by combining the determined respective probabilities of obtaining the measurements in the envelope data set for said pair of values of the systolic blood pressure and diastolic blood pressure. This embodiment provides that the systolic and diastolic blood pressure is determined according to the joint probability of each measurement in the envelope data set occurring.

In these embodiments, combining the respective probabilities may comprise multiplying the respective probabilities together or summing logarithms of the respective probabilities. The embodiment involving the summing of logarithms has advantages when identifying the systolic and diastolic blood pressure values as those that have the highest sum.

In some embodiments, the step of using the set of PDFs comprises determining respective likelihoods for obtaining the measurements in the envelope data set for all possible pairs of values of the systolic blood pressure and diastolic blood pressure. This embodiment has the advantage that an optimisation algorithm is not required to determine the systolic and diastolic blood pressure values, and the exhaustive search of all possible pairs of systolic and diastolic blood pressure values can enable a quantification of the quality of the determined systolic and diastolic blood pressure value, for example by determining the prominence of the highest probability.

In alternative embodiments, the step of using the set of PDFs and determining the systolic blood pressure and diastolic blood pressure comprises using an iterative optimisation algorithm to determine the highest likelihood for the envelope data set. These embodiments have the advantage that the systolic and diastolic blood pressure values can be determined more quickly and/or efficiently than with a full search across all possible combinations of systolic and diastolic blood pressure.

In some embodiments, the step of using the set of PDFs comprises determining a blood flow scaling factor based on the one or more highest measurements of blood flow in the envelope data set; and using the blood flow scaling factor to align blood flow values in the set of PDFs and the measurements of blood flow in the envelope data set. These embodiments enable the set of PDFs to be normalised to the envelope data set or the envelope data set to be normalised to the set of PDFs. In these embodiments, the step of determining the blood flow scaling factor may comprise determining the blood flow scaling factor based on a function or median average of a plurality of the highest measurements of blood flow in the envelope data set. The use of multiple measurements of blood flow to determine the blood flow scaling factor makes scaling less sensitive to outlier measurements and other errors.

In some embodiments, the method further comprises generating the set of PDFs. In these embodiments, the step of generating the set of PDFs may comprise generating the set of PDFs from a prior distribution relating to systolic blood pressure and diastolic blood pressure. In these embodiments, the prior distribution may comprise, or be based on, one or more of the following: information on physiologically possible values of systolic blood pressure and diastolic blood pressure; information on physiologically possible differences in systolic blood pressure and diastolic blood pressure values; one or more previous values of systolic blood pressure and diastolic blood pressure for the subject; one or more characteristics of the subject; one or more characteristics of a sensor used to measure the blood flow; and one or more characteristics of a device used to apply restriction to the body part. In this way, the PDFs can be constructed so that there is a relatively low likelihood of the subject being considered to have a systolic and diastolic blood pressure outside the usual physiological ranges for the subject or a population, unless it is strongly supported by the measured envelope data set.

In some embodiments, the set of PDFs comprise PDFs scaled by a PDF scaling factor.

In some embodiments, the different degrees of restriction are applied to the body part using a cuff that is inflated and/or deflated to a plurality of different pressures, or continuously inflated or deflated. In these embodiments, the envelope data set may be a set of measurement pairs, wherein a measurement pair comprises a measurement of blood flow in the body part and the respective pressure in the cuff when the measurement of blood flow is made. In these embodiments, the measurements of blood flow may be amplitudes of pressure oscillations in the cuff or measurements of pressure oscillations in a fluid or gel-filled pad beneath the cuff. Alternatively, in these embodiments the measurements of blood flow may be obtained from a photoplethysmography, PPG, signal from a PPG sensor positioned on the body part distal to the cuff. Alternatively, in these embodiments the measurements of blood flow may be obtained from an audio signal from an audio sensor or from an ultrasound sensor positioned on the body part distal to the cuff.

According to a second aspect, there is provided a computer program product comprising a computer readable medium having computer readable code embodied therein, the computer readable code being configured such that, on execution by a suitable computer or processing unit, the computer or processing unit is caused to perform the method according to the first aspect or any embodiment thereof. According to a third specific aspect, there is provided an apparatus configured to determine blood pressure of a subject. The apparatus comprises a processing unit configured to receive an envelope data set for the subject, the envelope data set comprising measurements of blood flow in a body part of the subject for respective different degrees of restriction applied to the body part; use a set of probability density functions, PDFs, to determine respective likelihoods for obtaining the measurements in the envelope data set for different pairs of values of the systolic blood pressure and diastolic blood pressure; and determine the systolic blood pressure and the diastolic blood pressure for the subject as the pair of values of the systolic blood pressure and diastolic blood pressure that provides the highest likelihood for the envelope data set. Thus, the third aspect provides an apparatus for determining systolic blood pressure and diastolic blood pressure measurement that is less reliant on particular measurement points or a single measurement point in the envelope data set. As a result, the apparatus is less susceptible to errors arising from outlier measurements and other errors in the envelope data set.

In some embodiments, the processing unit is configured to use the set of PDFs by, for each measurement in the envelope data set, determining respective probabilities for obtaining said measurement in the envelope data set for different pairs of values of the systolic blood pressure and diastolic blood pressure; and, for each pair of values of the systolic blood pressure and diastolic blood pressure, determining the likelihood for obtaining the measurements in the envelope data set by combining the determined respective probabilities of obtaining the measurements in the envelope data set for said pair of values of the systolic blood pressure and diastolic blood pressure. This embodiment provides that the systolic and diastolic blood pressure is determined according to the joint probability of each measurement in the envelope data set occurring.

In these embodiments, the processing unit can be configured to combine the respective probabilities by multiplying the respective probabilities together or by summing logarithms of the respective probabilities. The embodiment involving the summing of logarithms has advantages when identifying the systolic and diastolic blood pressure values as those that have the highest sum. In some embodiments, the processing unit is configured to use the set of PDFs by determining respective likelihoods for obtaining the measurements in the envelope data set for all possible pairs of values of the systolic blood pressure and diastolic blood pressure. This embodiment has the advantage that an optimisation algorithm is not required to determine the systolic and diastolic blood pressure values, and the exhaustive search of all possible pairs of systolic and diastolic blood pressure values can enable a quantification of the quality of the determined systolic and diastolic blood pressure value, for example by determining the prominence of the highest probability.

In alternative embodiments, the processing unit is configured to use the set of PDFs and determine the systolic blood pressure and diastolic blood pressure by using an iterative optimisation algorithm to determine the highest likelihood for the envelope data set. These embodiments have the advantage that the systolic and diastolic blood pressure values can be determined more quickly and/or efficiently than with a full search across all possible combinations of systolic and diastolic blood pressure.

In some embodiments, the processing unit is configured to use the set of PDFs by determining a blood flow scaling factor based on the one or more highest measurements of blood flow in the envelope data set; and using the blood flow scaling factor to align blood flow values in the set of PDFs and the measurements of blood flow in the envelope data set. These embodiments enable the set of PDFs to be normalised to the envelope data set or the envelope data set to be normalised to the set of PDFs. In these embodiments, the processing unit can be configured to determine the blood flow scaling factor based on a function or median average of a plurality of the highest measurements of blood flow in the envelope data set. The use of multiple measurements of blood flow to determine the blood flow scaling factor makes scaling less sensitive to outlier measurements and other errors.

In some embodiments, the processing unit is further configured to generate the set of PDFs. In these embodiments, the processing unit can be configured to generate the set of PDFs by generating the set of PDFs from a prior distribution relating to systolic blood pressure and diastolic blood pressure. In these embodiments, the prior distribution may comprise, or be based on, one or more of the following: information on physiologically possible values of systolic blood pressure and diastolic blood pressure; information on physiologically possible differences in systolic blood pressure and diastolic blood pressure values; one or more previous values of systolic blood pressure and diastolic blood pressure for the subject; one or more characteristics of the subject; one or more characteristics of a sensor used to measure the blood flow; and one or more characteristics of a device used to apply restriction to the body part. In this way, the PDFs can be constructed so that there is a relatively low likelihood of the subject being considered to have a systolic and diastolic blood pressure outside the usual physiological ranges for the subject or a population, unless it is strongly supported by the measured envelope data set.

In some embodiments, the set of PDFs comprise PDFs scaled by a PDF scaling factor.

In some embodiments, the different degrees of restriction are applied to the body part using a cuff that is inflated and/or deflated to a plurality of different pressures, or continuously inflated or deflated. In these embodiments, the envelope data set may be a set of measurement pairs, wherein a measurement pair comprises a measurement of blood flow in the body part and the respective pressure in the cuff when the measurement of blood flow is made. In these embodiments, the measurements of blood flow may be amplitudes of pressure oscillations in the cuff or measurements of pressure oscillations in a fluid or gel-filled pad beneath the cuff. Alternatively, in these embodiments the measurements of blood flow may be obtained from a photoplethysmography, PPG, signal from a PPG sensor positioned on the body part distal to the cuff. Alternatively, in these embodiments the measurements of blood flow may be obtained from an audio signal from an audio sensor or from an ultrasound sensor positioned on the body part distal to the cuff.

In some embodiments, the processing unit is configured to receive the envelope data set from a pressure sensor that measures the pressure in a cuff. In these embodiments, the apparatus may further comprise the pressure sensor, or the pressure sensor may be separate from the apparatus. In alternative embodiments, the processing unit is configured to receive measurements of the degree of restriction applied to the body part from a pressure sensor that measures pressure in a cuff, and to receive measurements of blood flow in the body part from a blood flow sensor, such as a PPG sensor, an audio sensor or an ultrasound sensor. In these embodiments, the apparatus may further comprise the pressure sensor and/or the blood flow sensor, or the pressure sensor and/or the blood flow sensor may be separate from the apparatus.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a graph illustrating an exemplary envelope data set;
Fig. 2 is a block diagram of an apparatus that can be used to implement the techniques described herein;
Fig. 3 is a flow chart illustrating a method of determining blood pressure according to various embodiments;
Fig. 4 is an illustration of a set of probability density functions (PDFs) for the normalised oscillation amplitude over a range of cuff pressure values and for a fixed systolic blood pressure and fixed diastolic blood pressure; and
Fig. 5 is an illustration of a map showing the likelihood of obtaining an envelope data set for different combinations of systolic blood pressure and diastolic blood pressure.

DETAILED DESCRIPTION OF EMBODIMENTS

As noted above, the disclosed techniques enable systolic blood pressure and diastolic blood pressure to be determined from an envelope data set with less reliance on particular measurement points or a single measurement point in the envelope data set than conventional techniques.

The envelope data set comprises measurements of blood flow in a body part for respective different degrees of restriction applied to the body part. For example, when a cuff is used to apply pressure to an upper arm of a subject, the blood flow is measured downstream of the cuff, i.e. in part of the arm distal to the cuff (e.g. in the lower arm or wrist). The envelope data set is therefore made up of a series of 'measurement pairs' or 'measurement points', with each pair including a measurement of the blood flow and a measurement of the pressure applied when that blood flow measurement was obtained (e.g. the static pressure in a cuff that is restricting the blood flow). In the case of an oscillometric measurement, the envelope data set would comprise pairs of static cuff pressure and cuff pressure oscillation amplitude (static cuff pressure, cuff pressure oscillation amplitude). The measurement set of value pairs is referred to herein as an "envelope data set" or a "measured envelope". The form of the blood flow measurements can depend on the type of sensor used to measure blood flow. In particular, the sensor should measure pulsatile blood flow. For example, the measurements of blood flow can be obtained by a sensor that measures or detects volume changes (such as plethysmography, including photoplethysmography (PPG), oscillometry or pressure-sensing using a fluid or gel-filled pad placed between an inflatable cuff and the body part), or by a sensor that measures flow velocity (such as Doppler ultrasound, or, to some extent, the acoustic detection of Korotkoff sounds).

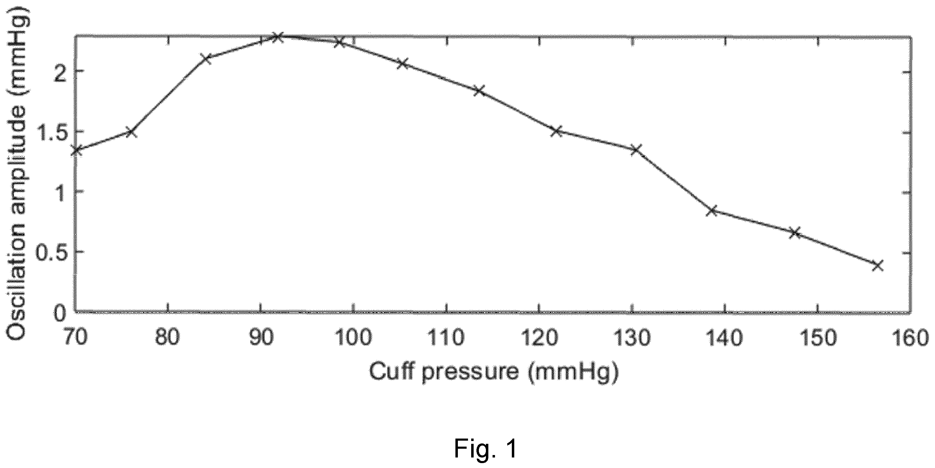

The graph in Fig. 1 shows part of an exemplary envelope data set comprising measurements of oscillation amplitude in mmHg against cuff pressure (also in mmHg) for a series of cuff pressures in the range 70 mmHg to 160 mmHg. The subject had a systolic blood pressure of 132 mmHg and a diastolic blood pressure of 74 mmHg as measured using a reference blood pressure measurement technique. It will be appreciated that an envelope data set can also include measurements of blood flow at cuff pressures outside the range shown in Fig. 1.

The techniques for determining/estimating the systolic and diastolic blood pressure consider the measurement pairs in the envelope data set to be samples of a probability distribution that depends on the variables "systolic blood pressure" and "diastolic blood pressure". Using the available measurement pairs in the envelope data set, and optionally, any prior information about the distribution of systolic and diastolic blood pressure, the techniques find the values for systolic and diastolic blood pressure that maximise the likelihood of observing the measured envelope data set.

In more detail, given a set of individual observations $x_i$ as $x$ corresponding to the measurement pairs in the envelope data set, the method finds an estimate $\hat{\theta}$ of the vector of population parameters $\theta$ (corresponding to combinations of systolic and diastolic blood pressure) from a sampling distribution $f$ and optionally the prior distribution $g(\theta)$, by finding the value of $\theta$ which maximizes the joint probability of the observations x:

$$\hat{\theta}(x) = \arg\max_{\theta} f(\theta|x) = \arg\max_{\theta} f(x|\theta)g(\theta) \qquad (1)$$

The joint probability can be calculated as the product of individual probabilities. However, certain embodiments can take into account that the measurements in the envelope data set are not statistically independent, in which case calculation the joint probability can be more involved than just multiplying the individual probabilities. In this case, instead of multiplying the probabilities, the maximum can also be found by maximising the sum of the logarithms of the individual probabilities, which avoids a long sequence of multiplications that may not be well-behaved numerically:

$$\hat{\theta}(x) = \arg\max_{\theta} f(\theta|x) = \arg\max_{\theta} f(x|\theta)g(\theta) =$$

$$\arg\max_{\theta} \prod_{i=1}^{N} f(x_i|\theta)g(\theta) = \arg\max_{\theta} \sum_{i=1}^{N} \log f(x_i|\theta)g(\theta)$$

$$(2)$$

Heuristically speaking, $f$ should describe the relationship that no or faintly pulsatile blood flow is more likely if the cuff pressure is outside the diastolic-systolic range, and highly pulsatile flow is more likely inside the diastolic-systolic range. In some preferred embodiments, $f$ can be constructed as a composition of smooth functions like the logistic function or bell curves, as this facilitates the use of standard optimisation algorithms to find the maximum. In some embodiments, the construction of $f$ also takes into account some information taken from the envelope data set itself, such as the range of minimum and maximum oscillation amplitude.

Once $f$ is constructed, its global maximum on the set of valid systolic and diastolic pairs can be found using one of several possible methods. As the set of possible systolic and diastolic blood pressure value pairs is of limited size, some embodiments provide that the full set can be searched exhaustively even with moderate computing power by calculating the likelihood value for every possible pair and finding the maximum value. It is also possible to use standard optimisation algorithms like inner-point methods or gradient search. Lastly, depending on the choice of $f$, it can be possible to use an analytic solution.

Thus, the presented techniques place as little reliance on individual measurement points in the envelope data set as possible during the calculation of the systolic blood pressure and the diastolic blood pressure. Additionally, the techniques efficiently use more or all of the available information, which includes measurement pairs in the envelope data set with no or insignificant blood flow measurements (e.g. no or insignificant oscillation amplitudes). The information contained in these measurement points is usually discarded in "maximum amplitude algorithm"-type approaches, but in the disclosed techniques, such measurement points will contribute information and make the maximum of the likelihood function more prominent. For example, a measurement pair in the envelope data set with a near-zero amplitude at cuff pressure X may make blood pressure ranges that do not include X more likely, and blood pressure ranges that do include X less likely.

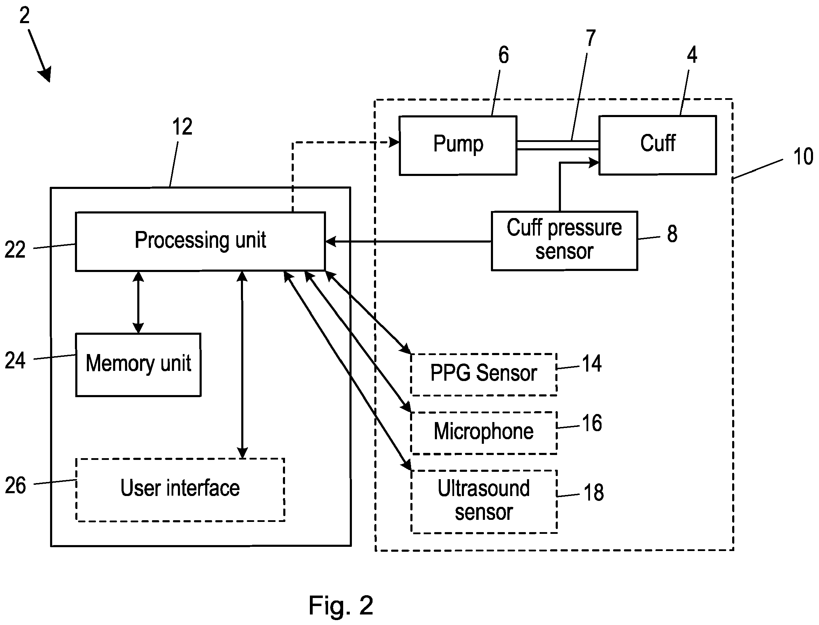

Fig. 2 is a block diagram of a system 2 according to various embodiments for determining the blood pressure of a subject according to the techniques described herein. The system 2 forms a non-invasive blood pressure (NIBP) monitoring system 2, i.e. a system that measures blood pressure of a subject non-invasively. As noted above, the blood pressure is determined from an envelope data set that comprises measurements of blood flow in a body part of the subject for respective different degrees of restrictions applied to the body part. In the embodiment illustrated in Fig. 2, a cuff 4 is used to apply the restriction to the body part. However, it will be appreciated that other types of devices can be used to apply a restriction to the body part to change the flow of blood in the body part. For example, a tourniquet can be used whose tightness is actuated electrically, mechanically, pneumatically or hydraulically to change the applied pressure.

The system 2 in Fig. 2 comprises a cuff 4, a pump 6 that is connected to the cuff 4 (e.g. via a connecting tube 7) and a cuff pressure sensor 8 for measuring the pressure in the cuff 4. The cuff pressure sensor 8 outputs a cuff pressure signal representing, or relating to, the pressure in the cuff 4 over time. The cuff pressure signal provides the measurements of the degree of restriction applied to the body part in the envelope data set. The cuff 4 is to be placed around a body part of a subject of interest, e.g. around a limb such as an arm or leg, and the pump 6 is controllable to selectively inflate the cuff 4. The pump 6 may also be able to selectively deflate the cuff 4, and/or a valve (not shown) may be provided for enabling the cuff 4 to be deflated. The cuff pressure sensor 8 measures the pressure in the cuff 4, at least when the cuff 4 is applying a defined stimulus on the artery located under the cuff 4 (this can be during inflation of the cuff 4, deflation of the cuff 4, or while the pressure in the cuff 4 is held at a particular level).

The cuff 4, pump 6 and cuff pressure sensor 8 can be considered to form a measurement device 10. In addition to the measurement device 10, the system 2 illustrated in Fig. 1 also comprises an apparatus 12 that operates according to the techniques described herein to determine the blood pressure of the subject from an envelope data set provided by the measurement device 10. Thus, the apparatus 12 is configured to receive the cuff pressure signal from the cuff pressure sensor 8. In some embodiments, the apparatus 12 is configured to control the operation of the pump 6, and thereby initiate the inflation/deflation of the cuff 4 at an appropriate time. Although the apparatus 12 is shown as being separate to the measurement device 10 in Fig. 2, it will be appreciated that in some implementations the measurement device 10 can be part of the apparatus 12, or vice versa.

The apparatus 12 may be in the form of, or be part of, a computing device, such as a server, desktop computer, laptop, tablet computer, smartphone, smartwatch, etc., or other types of device typically found in clinical environments, such as a patient monitoring device (e.g. a monitoring device located at the bedside of a patient in a clinical environment) that is used to monitor (and optionally display) various physiological characteristics of a subject/patient.

The system 2 comprises a blood flow sensor for measuring the blood flow (or pulsatile blood flow) in a body part of the subject. In some embodiments, the measurements of blood flow in the body part that form the envelope data set are measurements of the amplitude of pressure oscillations in the cuff 4 due to the flow of blood. In these embodiments, the measurements of blood flow are obtained from the cuff pressure signal, and so the cuff pressure sensor 8 is used as the blood flow sensor. This type of measurement of blood flow is also referred to as oscillometric measurements or oscillometry.

In alternative embodiments, the measurements of blood flow in the body part that form the envelope data set are obtained using a blood flow sensor in the form of a PPG sensor 14 that provides a PPG signal. This type of measurement of blood flow is also referred to as a photoplethysmographic measurement. Thus, in these embodiments, the system 2 comprises the cuff pressure sensor 8 and one or more PPG sensors 14. The one or more PPG sensors 14 can be part of the measurement device 10. A PPG sensor 14 is to be placed on the body of the subject distal to the cuff 4 and outputs a PPG signal that is related to the flow of blood passing through that part of the body. As known to those skilled in the art, a PPG sensor 14 comprises one or more light sensors, and typically one or more light sources. The PPG signal output by the PPG sensor(s) 14 may be a raw measurement signal from the light sensor, e.g. the PPG signal can be an analog or digital signal representing light intensity over time.

In other alternative embodiments, the measurements of blood flow in the body part that form the envelope data set are obtained using a blood flow sensor in the form of pressure sensor that measures the pressure or pressure oscillations in a fluid or gel-filled pad placed between the inflatable cuff 4 and the body part. This type of sensor provides a hydraulic coupling between the body part and the cuff 4, and provides a higher-quality signal than a pneumatic coupling between a body part and an air-filled cuff 4. The pressure sensor used to measure the pressure/pressure oscillations in the pad can be of the type that is usually used to invasively measure pressure in an artery. An example of such a blood flow sensor, which is also known as a 'shell cuff' is described in "Clinical Evaluation of a High-fidelity Upper Arm Cuff to Measure Arterial Blood Pressure during Noncardiac Surgery" by Josef Briegel, M.D. et al., Anesthesiology November 2020, vol. 133, 997-1006. The pressure sensor can be part of the measurement device 10. The pressure sensor can output a pressure signal that is related to the pressure in the pad due to pressure applied by the cuff 4 and the flow of blood passing through the part of the body beneath the pad and cuff 4. The pressure signal output by the pressure sensor may be a raw measurement signal from the pressure sensor, e.g. the pressure signal can be an analog or digital signal representing pressure/pressure oscillations in the pad over time.

In other alternative embodiments, the measurements of blood flow in the body part that form the envelope data set are obtained using a blood flow sensor in the form of one or more microphones 16 (for measuring the sound of the blood flow, including the Korotkoff sounds caused by turbulent flow in the arteries) or one or more ultrasound sensors 18 (for measuring blood flow velocity from a Doppler shift). This type of measurement of blood flow is also referred to as an auscultatory measurement. Thus, in these embodiments, the system 2 comprises the cuff pressure sensor 8 and one or more microphones 16 or ultrasound sensors 18. The one or more microphones 16 or one or more ultrasound sensors 18 can be part of the measurement device 10. The sensor(s) 16, 18 are to be placed on the body of the subject distal to the cuff 4 and output an audio signal that is related to the flow of blood passing through that part of the body. The audio signal output by the microphone(s) 16 or ultrasound sensor(s) 18 may be a raw measurement signal from the sensor(s) 16, 18, e.g. the audio signal can be an analog or digital signal representing sound/ultrasound over time.

Those skilled in the art will appreciate that measurements of blood flow in the body part can be obtained using other types of (non-invasive) sensors, and the disclosed techniques are not restricted to the sensors described above.

The apparatus 12 includes a processing unit 22 that controls the operation of the apparatus 12 and that can be configured to execute or perform the methods described herein. The processing unit 22 can be implemented in numerous ways, with software and/or hardware, to perform the various functions described herein. The processing unit 22 may comprise one or more microprocessors or digital signal processors (DSPs) that may be programmed using software or computer program code to perform the required functions and/or to control components of the processing unit 22 to effect the required functions. The processing unit 22 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 22 is connected to a memory unit 24 that can store data, information and/or signals for use by the processing unit 22 in controlling the operation of the apparatus 12 and/or in executing or performing the methods described herein. In some implementations the memory unit 24 stores computer-readable code that can be executed by the processing unit 22 so that the processing unit 22 performs one or more functions, including the methods described herein. In particular embodiments, the program code can be in the form of an application for a smartwatch, smartphone, tablet, laptop or computer. The memory unit 24 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 24 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 12 comprises a user interface 26 that includes one or more components that enables a user of apparatus 12 to input information, data and/or commands into the apparatus 12, and/or enables the apparatus 12 to output information or data to the user of the apparatus 12. Information that can be output by the user interface 26 can include the systolic blood pressure and diastolic blood pressure determined from the envelope data set according to the techniques described herein. The user interface 26 can comprise any suitable input component(s), including but not limited to a keyboard, keypad, one or more buttons, switches or dials, a mouse, a track pad, a touchscreen, a stylus, a camera, a microphone, etc., and/or the user interface 26 can comprise any suitable output component(s), including but not limited to a display screen, one or more lights or light elements, one or more loudspeakers, a vibrating element, etc.

It will be appreciated that a practical implementation of an apparatus 12 may include additional components to those shown in Fig. 2. For example, the apparatus 12 may also include a power supply, such as a battery, or components for enabling the apparatus 12 to be connected to a mains power supply. The apparatus 12 may also include interface circuitry for enabling a data connection to and/or data exchange with other devices, including any one or more of the measurement device 10 (or a separate cuff pressure sensor 8), sensors 14, 16, 18, servers, databases and user devices.

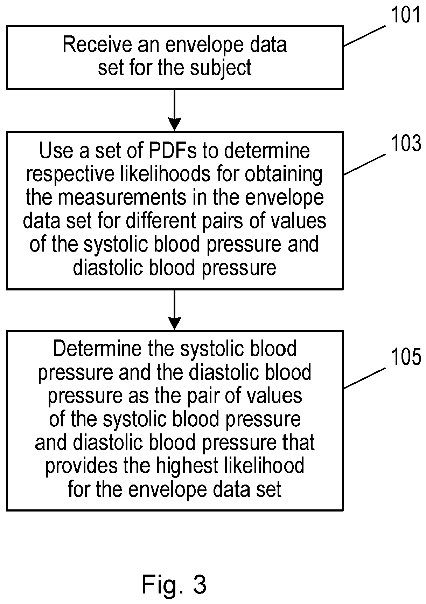

The flow chart in Fig. 3 illustrates a method of determining blood pressure according to embodiments of the techniques described herein. Various steps of the method can be performed by the apparatus 12, and in particular the processing unit 22. In that respect, computer program code can be provided that causes the processing unit 22, and thus the apparatus 12, to perform the method described below when that code is executed by the processing unit 22. In general, the method in Fig. 3 can be implemented by a processor, processing unit or a computer. As noted further below, one or more of the steps of the method in Fig. 3 can make use of or involve use of any of the cuff 4 (or other blood flow restriction device), the pump 6, the cuff pressure sensor 8 (or other 'degree of restriction' sensor and/or blood flow sensor), and (if present) PPG sensor 14, microphone 16 and ultrasound sensor 18.

In step 101 of the method in Fig. 3, an envelope data set for the subject is received. As noted above, the envelope data set comprises measurements of blood flow in a body part of the subject for respective different degrees of restriction applied to the body part. The envelope data set may be received in step 101 directly from the relevant sensor(s) 8, 14, 16, 18, for example as the blood flow measurements are obtained, in which case the envelope data set is formed from the received measurements. Alternatively, step 101 may comprise retrieving a previously obtained envelope data set from a memory, e.g. from memory unit 24.

In step 103, a set of probability density functions (PDFs) are used to determine respective likelihoods for obtaining the measurements in the envelope data set for different pairs of values of the systolic blood pressure and diastolic blood pressure. As noted above, the PDFs are used to quantify the likelihood of a specific envelope data set occurring for different pairs of values of the systolic blood pressure and the diastolic blood pressure. The PDFs in the set are a function of four variables: the measure of blood flow (e.g. oscillation amplitude), the degree of restriction (e.g. cuff pressure), systolic blood pressure and diastolic blood pressure. Since the measure of blood flow is the key unknown variable that is obtained by measurement, the PDFs are used to quantify the likelihood that a particular measure of blood flow at a particular degree of restriction corresponds to different systolic and diastolic blood pressures. Therefore individual PDFs in the set of PDFs can be considered as being for a particular value for the degree of restriction, and a particular pair of values of systolic blood pressure and diastolic blood pressure.

Using the terminology in equations (1) and (2), the set of PDFs quantify how likely it is to observe the combination of the set of measurement pairs x forming the envelope data set and the parameter vector $\theta$ (corresponding to combinations of systolic and diastolic blood pressure). The set of PDFs can be considered to be formed of a series of subsets of PDFs, with each subset of PDFs corresponding to a particular pair of values of systolic blood pressure and diastolic blood pressure (e.g. systolic blood pressure of 120 mmHg and diastolic blood pressure of 80 mmHg), and each PDF in the subset being a PDF for that pair of systolic blood pressure and diastolic blood pressure values and a particular cuff pressure value (e.g. 100 mmHg).

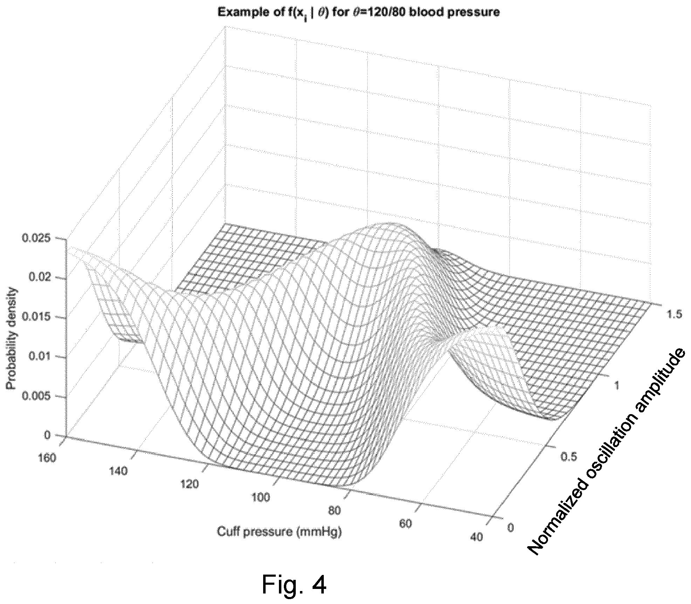

An exemplary subset of PDFs, $f(x_i|\theta)$, for a fixed value of systolic blood pressure of 120 mmHg and a fixed value of diastolic blood pressure of 80 mmHg is shown in Fig. 4. This subset of PDFs comprises a respective PDF for each cuff pressure value. Based on this exemplary subset of PDFs, measuring a low (or zero) oscillation amplitude far outside the cuff pressure interval of 80 mmHg to 120 mmHg is very probable, and measuring a high oscillation amplitude is very improbable. The reverse is true for cuff pressures inside the interval of 80 mmHg to 120 mmHg. Normalisation is discussed further below, and in some embodiments normalisation of the oscillation amplitudes is performed using a plurality of the highest magnitude oscillation amplitudes, e.g. a median value of the plurality of the highest N magnitude oscillation amplitudes. Consequently, the normalisation is not based on solely the maximum observed oscillation amplitude, and so the maximum observed oscillation amplitude will be normalised to a value above 1. This is taken into account by also having the set of PDFs cover normalised amplitudes greater than 1. In a 'good quality' measurement, there will usually be another envelope point close to the maximum. Therefore, even when normalising using a median value, the maximum oscillation amplitude will be close to 1. However, in measurements that include artefacts, the maximum may be an outlier and have a value much larger than 1 after normalisation. This large value is equally improbable for any combination of systolic/diastolic pressure and hence will have very little influence on the position of the likelihood maximum.

Thus, in step 103, the set of PDFs is used to determine the likelihood of a pair of values of the systolic blood pressure and the diastolic blood pressure for the received envelope data set. In other words, step 103 provides the likelihoods for the subject having different pairs of values of the systolic blood pressure and the diastolic blood pressure given the received envelope data set. The respective likelihoods determined in step 103 are also referred to herein as 'joint likelihoods' since they represent the likelihood of all the measurement pairs in the envelope data set occurring for a given pair of values of the systolic blood pressure and diastolic blood pressure.

Particular embodiments of step 103 comprise a two-step process to determine the respective likelihoods. In the first step, for each measurement pair in the envelope data set, respective probabilities for obtaining the measurement pair in the envelope data set for different pairs of values of the systolic blood pressure and diastolic blood pressure are determined. That is, for each measurement pair, a first probability is determined for a first pair of values of systolic blood pressure and diastolic blood pressure, a second probability is determined for a second pair of values of systolic blood pressure and diastolic blood pressure, and so on. In the second step, for each pair of values of the systolic blood pressure and diastolic blood pressure evaluated in the first step, the likelihood of obtaining the envelope data set is determined by combining the probabilities for that pair of values of the systolic blood pressure and diastolic blood pressure determined in the first step. In the second step the probabilities can be combined by multiplying the probabilities together, or by summing logarithms of the respective probabilities. Summing logarithms of the respective probabilities can be preferred, as this avoids a possibly numerically poorly behaved sequence of multiplications that can occur when multiplying the probabilities.

In some embodiments, step 103 can comprise determining respective likelihoods for the envelope data set for all possible pairs of values of the systolic blood pressure and diastolic blood pressure. That is, all possible combinations of systolic blood pressure and diastolic blood pressure are evaluated in step 103. This exhaustive search is possible since the size of the set of pairs of systolic and diastolic blood pressure values is relatively small.

In alternative embodiments, step 103 can comprise applying an iterative optimisation algorithm to determine the respective likelihoods. These embodiments can typically be used where steps 103 and 105 (described below) are performed in parallel or otherwise at the same time. In these embodiments, it may not be necessary to perform an exhaustive search and determine the likelihoods for all possible pairs of systolic and diastolic blood pressure.

Returning to the exemplary subset of PDFs in Fig. 4, measurement pairs with a high probability under this distribution would increase the likelihood of a blood pressure of 120/80 mmHg producing the set of measured envelope points, while measurement pairs with a low probability (e.g. low oscillation amplitude at 100 mmHg cuff pressure, or high amplitude at 150 mmHg cuff pressure) would decrease the likelihood of a blood pressure of 120/80 mmHg producing the measurement pairs in the envelope data set.

Once the respective likelihoods have been determined in step 103, in step 105 the systolic blood pressure and the diastolic blood pressure for the subject is determined as the pair of values of the systolic blood pressure and diastolic blood pressure that provides the highest likelihood for the envelope data set.

In embodiments of step 103 where an exhaustive search is performed of the possible pairs of systolic and diastolic blood pressure, step 105 can comprise determining the systolic and diastolic blood pressure of the subject as the pair of systolic blood pressure and diastolic blood pressure that provided the highest likelihood for the received envelope data set. In embodiments of step 103 where an iterative optimisation algorithm is used to determine the respective likelihoods, the algorithm can iterate through pairs of values of systolic and diastolic blood pressure to find a maximum in the likelihood for the envelope data set. Those skilled in the art will be aware of various different iterative optimisation algorithms that can be used to implement steps 103/105, and details are not provided herein. Nevertheless, techniques such as sequential quadratic programming, the interior-point method or the active-set method could be used in step 103/105. Depending on the way the likelihood function is constructed, there may be more than one local maximum. In this case, a single run of a local optimisation algorithm may arrive at one of the local maxima instead of the global one. Global optimisation algorithms could also be used.

In embodiments where an exhaustive search, or a substantially exhaustive search (e.g. over all plausible combinations of values of systolic blood pressure and diastolic blood pressure), is performed, the method can further comprise determining the prominence or significance of the maximum likelihood. The prominence or significance can be determined by examining a neighbourhood of a defined size around the maximum. The algorithm could calculate the average or minimum likelihood in the neighbourhood, and calculate the ratio of the maximum and this value. If the ratio is large, then then the maximum is considered prominent. If the ratio is small, then the maximum is not very distinct/prominent/significant. The prominence or significance of the maximum can provide a quantification of the quality of the determined systolic and diastolic blood pressure values. In some embodiments, if the received envelope data set does not provide a maximum that is sufficiently prominent or significant, the values of systolic and diastolic blood pressure can be discarded as being potentially unreliable or not sufficiently accurate. The exact definition of prominence can depend on the functions used to construct the likelihood function. Numerical values could be determined by simulation (e.g. using synthetic, generated envelopes as inputs which represent both known good and poor envelopes), as well as empirically (e.g. using real envelopes that are known to be either good or poor). Statistical methods including machine learning can then be used to determine the optimal thresholds for discarding/retaining a value of systolic blood pressure/diastolic blood pressure.

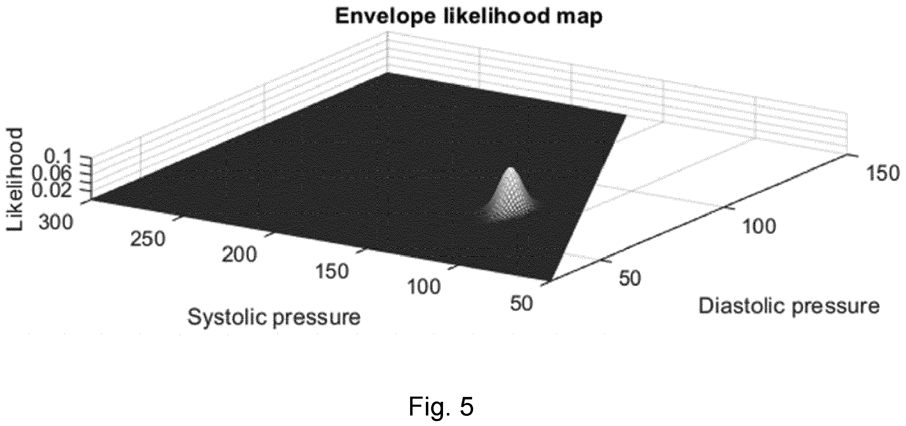

Fig. 5 is an illustration of a map showing the likelihood of obtaining the envelope data set shown in Fig. 1 for different combinations of systolic blood pressure and diastolic blood pressure. The likelihoods shown in Fig. 5 are log likelihoods for the envelope data set shown in Fig. 1. Fig. 5 therefore represents the output of an exhaustive search of all possible pairs of systolic and diastolic blood pressure in step 103 for the envelope data set in Fig. 1. It can be seen in Fig. 5 that the maximum likelihood for this envelope data set occurs at a systolic blood pressure of 132 mmHg and a diastolic blood pressure of 74 mmHg, with the determined likelihood falling off significantly (even to 0) at just small differences in these values (e.g. the likelihood is approximately 0 outside the systolic blood pressure range of 110-140 mmHg and approximately 0 outside the diastolic blood pressure range of 60-80 mmHg.

Although not shown in Fig. 3, the values of the systolic blood pressure and/or diastolic blood pressure determined in step 105 can be output, for example visually and/or audibly to the subject or another user of the method/apparatus 12, such as a physician or other healthcare provider, and/or output electronically, for example in the form of a signal that is sent to a device or apparatus that stores the values of the systolic blood pressure and/or diastolic blood pressure in a patient record for the subject. Each PDF in the set of PDFs can be formed from smooth functions, such as the logistic function and/or the Gaussian bell curve. The use of smooth functions can facilitate the use of iterative optimisation algorithms for finding the maximum likelihood. In preferred embodiments, the set of PDFs can be constructed such that certain combinations of systolic blood pressure and diastolic blood pressure have a zero probability associated with them. For example, all pairs of systolic blood pressure and diastolic blood pressure where the diastolic blood pressure is higher than the systolic blood pressure should have a probability of zero (by definition of systolic blood pressure and diastolic blood pressure).

The exemplary subset of PDFs in Fig. 4 was generated by approximating the expected oscillation amplitude as a Gaussian bell curve with cuff pressure being the parameter. When following the cuff pressure axis, the position of the maximum on the oscillation amplitude axis describes this bell curve. To define the set of probability density functions, a family of bell curves is used that have their mean value at the maximum, which each bell curve in the family corresponding to a particular pair of values of systolic and diastolic blood pressure. Each individual curve is scaled so that the area under the curve is one (since the real Gaussian bell curve is defined over the real number, but the pressure and oscillation amplitude cannot be negative). Although this is a rough approximation, even in this form it is a suitable detector for envelope data sets resulting from 120/80 blood pressure.

As an alternative to approximating the shape of the envelope as a Gaussian bell curve, other bell-shaped functions could be used, including the extreme (and non-smooth) case of a function that is 0 outside the diastolic/systolic interval and 1 inside it. This will still result in a detector for the given blood pressure.

To form a subset of probability density functions, bell-shaped distributions can be used in the diastolic/systolic interval, while outside this interval, either scaled bell-shaped distributions, or one-sided distributions like the exponential distribution, can be used.

It will be appreciated that the functions used to form the PDFs in the set in a particular implementation can be chosen as a compromise between computational complexity and modelling accuracy. A set of simple base functions that work "well enough" may be preferable to a computationally complex model that provides only minimal additional benefits.

In some embodiments, step 103 comprises scaling the envelope data set or the set of PDFs based on the envelope data set. This scaling is performed using a scaling factor referred to as a "blood flow scaling factor". The blood flow scaling factor can be used to align the blood flow values in the set of PDFs and the measurements of blood flow in the envelope data set. Thus, the blood flow scaling factor can be used to normalise the blood flow values of each PDF to the envelope data set, or to normalise the blood flow measurements in the envelope data set. Alternatively, the blood flow scaling factor can be used to align the blood flow values in the set of PDFs and the measurements of blood flow in the envelope data set by distributing the blood flow scaling factor across both the blood flow values in the set of PDFs and the measurements of blood flow in the envelope data set. For example, the blood flow scaling factor can be distributed by multiplying one of (i) the blood flow values in the set of PDFs, and (ii) the measurements of blood flow in the envelope data set, by the square root of the blood flow scaling factor, and dividing the other one of (i) the blood flow values in the set of PDFs and (ii) the measurements of blood flow in the envelope data set by the square root of the blood flow scaling factor. The blood flow scaling factor can be based on the one or more highest measurements of blood flow in the envelope data set. Typically, the magnitude of the highest measurements of blood flow in an envelope data set depends on a number of factors, such as the physical characteristics of the subject, the cuff size, cuff type, how firmly the cuff is attached to the body part, etc., which can vary between subjects and vary between measurements for the same subject. Therefore, deriving a blood flow scaling factor for the PDFs using the magnitude of the highest measurements of blood flow enables the PDFs to be adapted to those factors. In some embodiments, the blood flow scaling factor is determined based on the measurement of the blood flow in the envelope data set with the highest magnitude. For example, the measurement of blood flow with the maximum amplitude can be scaled to 1, and all other amplitudes can be scaled accordingly. However, it is possible that the highest magnitude blood flow measurement is an artefact, leading to a significant error, so preferably the blood flow scaling factor is determined from a plurality of the highest magnitude measurements of blood flow to make the scaling less sensitive to outlier measurements and other errors. The blood flow scaling factor can be determined from a function of the plurality of highest magnitude measurements, and the function can be an average, such as mean, mode or median (with median being a more preferable option to mean and mode). The number of measurements to use to determine the blood flow scaling factor can be between 2 and 10, for example 3, 5 or 7. When taking the median and using the 3 highest magnitude measurements, the blood flow scaling factor is robust to one of the maxima being an artefact, when taking the median and using the 5 highest magnitude measurements, the blood flow scaling factor is robust to two of the maxima being an artefact, etc. Determining the blood flow scaling factor from a plurality of the highest magnitude blood flow measurements also contributes to the blood pressure measurement technique being less reliant on a single measurement point in the envelope data set.

In some embodiments, the method can further comprise the step of generating the set of PDFs. The set of PDFs can be generated as outlined above with respect to Fig. 4. As noted above, in some embodiments prior information about the distribution of systolic and diastolic blood pressure can be used or taken into account when generating the PDFs. The prior information may be in the form of one or more prior distributions, g(8). The prior information may be based on static information, i.e. information that does not change, or that does not change quickly. Static information can include, for example, characteristics of the subject (e.g. age, height, weight, medical conditions, medications, etc.), characteristics of the sensor used to measure blood flow (e.g. an indication of an offset and/or noise present in the measurements by that sensor), characteristics of the device (e.g. cuff) used to apply a restriction to the body part, information on physiologically possible values of systolic blood pressure and diastolic blood pressure, and information on physiologically possible differences in systolic blood pressure and diastolic blood pressure values. In the latter two examples (as noted above), certain combinations of systolic blood pressure and diastolic blood pressure have a zero probability of occurring, and other combinations of systolic blood pressure and diastolic blood pressure have a near-zero or otherwise low probability of occurring. For example, diastolic blood pressure cannot be higher than systolic blood pressure, so any pairs where diastolic blood pressure is higher than the systolic blood pressure should have a probability of zero. Certain values of systolic blood pressure and diastolic blood pressure at the extremes, e.g. very low values and very high values outside known physiological ranges, can also have a zero or near-zero probability. Likewise, certain values of pulse pressure (the difference between systolic blood pressure and diastolic blood pressure) are also less likely, and should have a near-zero or otherwise low probability of occurring. In a similar manner, certain values of systolic blood pressure and diastolic blood pressure are more likely, e.g. values within the typical physiological ranges, and can have probabilities in the PDFs that make these more likely. Likewise, certain values of pulse pressure are also more likely, and the PDFs should be constructed accordingly. With this prior information being taken into account in the PDFs, there is a relatively low likelihood of the subject being considered to have a systolic and diastolic blood pressure outside the usual physiological range unless it is strongly supported by the measured envelope data set.

In some embodiments, the prior information may also or alternatively comprise dynamic information, i.e. information that does change, or does change quickly. Dynamic information can include one or more previous values of systolic blood pressure and diastolic blood pressure for the subject. This dynamic information can be used to generate the PDFs such that it is more likely that the new values for systolic and diastolic blood pressure found using the envelope data set are close to the previous values of systolic and diastolic blood pressure for the subject. The exact effect of the previous values for the subject on the PDFs can depend on how recent the previous values are, since blood pressure changes relatively slowly, and so more recent previous values are more likely to be indicative of the current values for systolic and diastolic blood pressure than older previous values.

While a strict mathematical definition of a PDF requires the integral over the entire range to be 1, it will be appreciated that the above method can be implemented using PDFs that have been scaled by some PDF scaling factor so that the integral over the entire range is greater (or less than) 1, and using the set of PDFs in step 103 should be understood accordingly. That is, step 103 includes using a set of PDFs, or using a set of PDFs for which the probability axis has been scaled by a scaling factor. For example, referring to Fig 4, the probability density axis could be scaled such that the maximum probability density provided by the set of PDFs is 3. The PDF scaling factor may be arbitrary, e.g. 2, 3, etc., or it may be selected to improve the performance of an algorithm that implements steps 103 and 105.

Therefore there is provided techniques for determining systolic and diastolic blood pressure from an envelope data set with less reliance on particular measurement points or a single measurement point in the envelope data set than conventional techniques.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of determining blood pressure of a subject, the method comprising:

> receiving (101) an envelope data set for the subject, the envelope data set comprising measurements of blood flow in a body part of the subject for respective different degrees of restriction applied to the body part;

using (103) a set of probability density functions, PDFs, to determine respective likelihoods for obtaining the measurements in the envelope data set for different pairs of values of the systolic blood pressure and diastolic blood pressure; and

determining (105) the systolic blood pressure and the diastolic blood pressure for the subject as the pair of values of the systolic blood pressure and diastolic blood pressure that provides the highest likelihood for the envelope data set.

2. A method as claimed in claim 1, wherein the step of using the set of PDFs comprises determining respective likelihoods for obtaining the measurements in the envelope data set for all possible pairs of values of the systolic blood pressure and diastolic blood pressure.

3. A method as claimed in claim 1, wherein the step of using the set of PDFs and determining the systolic blood pressure and diastolic blood pressure comprises using an iterative optimisation algorithm to determine the highest likelihood for the envelope data set.

4. A method as claimed in any of claims 1-3, wherein the step of using the set of PDFs comprises:

determining a blood flow scaling factor based on the one or more highest measurements of blood flow in the envelope data set; and

using the blood flow scaling factor to align blood flow values in the set of PDFs and the measurements of blood flow in the envelope data set.

5. A method as claimed in claim 4, wherein the step of determining the blood flow scaling factor comprises determining the blood flow scaling factor based on a function or median average of a plurality of the highest measurements of blood flow in the envelope data set.

6. A method as claimed in any of claims 1-5, wherein the method further comprises:

generating the set of PDFs from a prior distribution relating to systolic blood pressure and diastolic blood pressure.

7. A method as claimed in claim 6, wherein the prior distribution comprises, or is based on, one or more of the following:

- information on physiologically possible values of systolic blood pressure and diastolic blood pressure;
- information on physiologically possible differences in systolic blood pressure and diastolic blood pressure values;
- one or more previous values of systolic blood pressure and diastolic blood pressure for the subject;
- one or more characteristics of the subject;
- one or more characteristics of a sensor used to measure the blood flow; and
- one or more characteristics of a device used to apply restriction to the body part.

8. A computer program product comprising a computer readable medium having computer readable code embodied therein, the computer readable code being configured such that, on execution by a suitable computer or processing unit, the computer or processing unit is caused to perform the method of any of claims 1-7.

9. An apparatus (12) configured to determine blood pressure of a subject, the apparatus (12) comprising a processing unit (22) that is configured to:

receive an envelope data set for the subject, the envelope data set comprising measurements of blood flow in a body part of the subject for respective different degrees of restriction applied to the body part;

use a set of probability density functions, PDFs, to determine respective likelihoods for obtaining the measurements in the envelope data set for different pairs of values of the systolic blood pressure and diastolic blood pressure; and

determine the systolic blood pressure and the diastolic blood pressure for the subject as the pair of values of the systolic blood pressure and diastolic blood pressure that provides the highest likelihood for the envelope data set.

10. An apparatus (12) as claimed in claim 9, wherein the processing unit (22) is configured to use the set of PDFs by determining respective likelihoods for obtaining the measurements in the envelope data set for all possible pairs of values of the systolic blood pressure and diastolic blood pressure.

11. An apparatus (12) as claimed in claim 9, wherein the processing unit (22) is configured to use the set of PDFs and determine the systolic blood pressure and diastolic blood pressure by using an iterative optimisation algorithm to determine the highest likelihood for the envelope data set.

12. An apparatus (12) as claimed in any of claims 9-11, wherein the processing unit (22) is configured to use the set of PDFs by:

determining a blood flow scaling factor based on the one or more highest measurements of blood flow in the envelope data set; and
using the blood flow scaling factor to align blood flow values in the set of PDFs and the measurements of blood flow in the envelope data set.

13. An apparatus (12) as claimed in claim 12, wherein the processing unit (22) is configured to determine the blood flow scaling factor based on a function or median average of a plurality of the highest measurements of blood flow in the envelope data set.

14. An apparatus (12) as claimed in any of claims 9-13, wherein the processing unit (22) is further configured to:
generate the set of PDFs from a prior distribution relating to systolic blood pressure and diastolic blood pressure.

15. An apparatus (12) as claimed in claim 14, wherein the prior distribution comprises, or is based on, one or more of the following:

- information on physiologically possible values of systolic blood pressure and diastolic blood pressure;
- information on physiologically possible differences in systolic blood pressure and diastolic blood pressure values;
- one or more previous values of systolic blood pressure and diastolic blood pressure for the subject;
- one or more characteristics of the subject;
- one or more characteristics of a sensor used to measure the blood flow; and
- one or more characteristics of a device used to apply restriction to the body part.

Fig. 1

EP 4 082 430 A1

Fig. 2

Receive an envelope data
set for the subject — 101

Use a set of PDFs to determine
respective likelihoods for obtaining
the measurements in the envelope
data set for different pairs of values
of the systolic blood pressure and
diastolic blood pressure — 103

Determine the systolic blood
pressure and the diastolic blood
pressure as the pair of values
of the systolic blood pressure
and diastolic blood pressure that
provides the highest likelihood
for the envelope data set — 105

Fig. 3

Example of $f(x_i \mid \theta)$ for $\theta$=120/80 blood pressure

Fig. 4

Envelope likelihood map

Fig. 5

Europäisches
Patentamt

European
Patent Office

Office européen
des brevets

## EUROPEAN SEARCH REPORT

Application Number

EP 21 17 1027

### DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | LEE SOOJEONG ET AL: "On Using Maximum a Posteriori Probability Based on a Bayesian Model for Oscillometric Blood Pressure Estimation", SENSORS, vol. 13, no. 10, 10 October 2013 (2013-10-10), pages 13609-13623, XP55841503, DOI: 10.3390/s131013609 Retrieved from the Internet: URL:https://www.ncbi.nlm.nih.gov/pmc/articles/PMC3859082/pdf/sensors-13-13609.pdf> * page 13610 - page 13616 * ----- | 1-15 | INV. A61B5/022 |
| A | US 2003/181816 A1 (SHIRASAKI OSAMU [JP]) 25 September 2003 (2003-09-25) * paragraphs [0063], [0085], [0087], [0095], [0104]; figures 1,6 * ----- | 1-15 | |

TECHNICAL FIELDS SEARCHED     (IPC)

A61B

The present search report has been drawn up for all claims

| Place of search | Date of completion of the search | Examiner |
|---|---|---|
| The Hague | 17 September 2021 | Lommel, André |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 17 1027

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2021

| Patent document cited in search report | Publication date | Patent family member(s) | Publication date |
|---|---|---|---|
| US 2003181816 A1 | 25-09-2003 | CN 1440722 A<br>DE 10308895 A1<br>JP 2003250770 A<br>US 2003181816 A1 | 10-09-2003<br>11-09-2003<br>09-09-2003<br>25-09-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4984577 A

- US 5704362 A

**Non-patent literature cited in the description**

- **JOSEF BRIEGEL, M.D. et al.** Clinical Evaluation of a High-fidelity Upper Arm Cuff to Measure Arterial Blood Pressure during Noncardiac Surgery. *Anesthesiology,* November 2020, vol. 133, 997-1006